# EUROPÄISCHE PATENTSCHRIFT

(11) **EP 1 430 932 B1**
(45) Veröffentlichungstag und Bekanntmachung des Hinweises auf die Patenterteilung: **13.02.2008**
(21) Anmeldenummer: 03028515.9
(22) Anmeldetag: 10.12.2003
(51) Int. Cl.: A61N 5/10, G21K 5/00

(54) **Ionenstrahlanlage**
Ion beam facility
Installation a faisceau ionique

(30) Priorität: 20.12.2002 DE 10261099
(43) Veröffentlichungstag der Anmeldung: 23.06.2004
(73) Patentinhaber: SIEMENS AKTIENGESELLSCHAFT, 80333 München (DE)
(72) Erfinder: Reimoser, Stefan, Dr., 82031 Grünwald (DE); Solbrig, Michael, 80337 München (DE)

(56) Entgegenhaltungen:
- EP-A- 0 986 071
- MIZOTA M ET AL: "THE HIGH-ENERGY BEAM-TRANSPORT SYSTEM FOR HIMAC" MITSUBISHI ELECTRIC ADVANCE, MITSUBISHI ELECTRIC CORPORATION, TOKYO, JP, Bd. 62, 1995, Seiten 2-4, XP000905407 ISSN: 0386-5096
- ARDUINI G ET AL: "An H/light ion synchrotron for radiation therapy" NUCLEAR INSTRUMENTS & METHODS IN PHYSICS RESEARCH, SECTION - A: ACCELERATORS, SPECTROMETERS, DETECTORS AND ASSOCIATED EQUIPMENT, NORTH-HOLLAND PUBLISHING COMPANY. AMSTERDAM, NL, Bd. A365, Nr. 2, 11. November 1995 (1995-11-11), Seiten 542-552, XP004009009 ISSN: 0168-9002
- BRAHME A ET AL: "Design of a centre for biologically optimised light ion therapy in Stockholm" NUCLEAR INSTRUMENTS & METHODS IN PHYSICS RESEARCH, SECTION - B: BEAM INTERACTIONS WITH MATERIALS AND ATOMS, NORTH-HOLLAND PUBLISHING COMPANY. AMSTERDAM, NL, Bd. 184, Nr. 4, Dezember 2001 (2001-12), Seiten 569-588, XP004313116 ISSN: 0168-583X
- AMALDI U: "Cancer therapy with particle accelerators" NUCLEAR PHYSICS, NORTH-HOLLAND, AMSTERDAM, NL, Bd. A654, Nr. 1-2, 26. Juli 1999 (1999-07-26), Seiten 375c-99c, XP002232420 ISSN: 0375-9474

## Beschreibung

Die Erfindung betrifft eine Ionenstrahlanlage.

Beispielsweise aus dem Artikel von U. Linz "Tumortherapie mit Ionenstrahlen", Spektrum der Wissenschaft, Dossier 1/1999, Seiten 42 bis 51, ist bekannt, dass schnelle Protonen oder andere geladene Teilchen ihre Energie, beispielsweise im menschlichen oder tierischen Körper konzentrierter als Röntgen- oder Gammaquanten abgeben. Dadurch eignen sie sich besonders zur Bekämpfung von Tumoren. Richtig gesteuert, schädigen sie hauptsächlich die Geschwulst, während das umgebende gesunde Gewebe geschont wird. Dies bringt wesentliche Vorteile wie geringere Nebenwirkungen, schnellere Heilung und weniger Spätkomplikationen. Für vorgenannte Tumortherapie sind dabei weltweit etwa zwanzig Therapiezentren bekannt, die in dem Artikel aufgelistet sind.

Ferner ist aus der DE 100 10 523 A1 für eine Ionenstrahlanlage, bei der ausgehend von einem Ionenstrahlerzeuger mehrere Bestrahlungsräume fächerartig angeordnet sind, wenigstens ausschnittsweise der zugehörige Grundriss bekannt.

Eine solche Ionenstrahlanlage wird auch in der Veröffentlichung "The high-energy beam transport system for HIMAC" (Mitsubishi Electric Advance ; Tokyo, Seiten 2-4, Bd. 62, 1995) von Mizota et al.

Eine Aufgabe der vorliegenden Erfindung ist es, eine verbesserte Ionenstrahlanlage zu schaffen, die unter anderem Erweiterungen und Umbauten mit möglichst kurzen Betriebsunterbrechungen der Ionenstrahlanlage ermöglicht.

Diese Aufgabe wird erfindungsgemäß durch die Gegenstände der Ansprüche 1, 2, 6 und 8 gelöst. Vorteilhafte Ausgestaltungen sind in den Unteransprüchen beschrieben.

Durch die für Öffnungen vorbereitete Wand gemäß Anspruch 1, insbesondere bei Ausführung der Wand als Strahlung dämmende Wand, können bei einer Erweiterung der, ein erstes Ionenstrahlsystem, beispielsweise für Wasserstoffionen, umfassenden Ionenstrahlanlage um eine zweites Ionenstrahlsystem, beispielsweise für Kohlenstoffionen, die Erweiterungsarbeiten zunächst ohne eine Betriebsunterbrechung des ersten Ionenstrahlsystems durchgeführt werden, und nach der Erweiterung können in einfacher und schneller Weise aufgrund der bereits in der Wand vorgesehenen Öffnungen auch Bestrahlungsplätze des ersten Ionenstrahlsystems für einen Mischbetrieb im Rahmen beider Ionenstrahlsysteme oder zum ausschließlichen Betrieb im Rahmen des zweiten Ionenstrahlsystems umgerüstet werden. Da das zweite Ionenstrahlsystem für die schwerere Ionenart durch entsprechende Umjustierungen auch mit der ersten Ionenart betreibbar ist, kann auch ausschließlich mit dem zweiten Ionenstrahlsystem ein sich abwechselnden Betrieb mit der einen oder anderen Ionenart erfolgen, wobei je Wechsel vorgenannte Umjustierungen durchzuführen sind. Dadurch kann in einer Ausführungsform sogar ganz auf den Ionenstrahlerzeuger und die Ionentransportstrecke des ersten Ionenstrahlsystems verzichtet werden, wobei dadurch gegenüber einem Mischbetrieb mit beiden Ionenstrahlsystemen natürlich der Vorteil wegfällt, dass im Falle einer Wartung des ersten oder zweiten Ionenstrahlsystems mit dem zweiten oder ersten Ionenstrahlsystem weiter Betrieb gemacht werden kann. Vorausgehend beschriebene Vorteile sind bei der Ionenstrahlanlage gemäß Anspruch 2 ebenfalls erzielbar, bei der entlang einer Wand eines Gebäudes der Ionenstrahlanlage eine Ionentransportstrecke des ersten Ionenstrahlsystems geführt, und hinter der Wand Platz für wenigstens eine nachinstallierbare zweite Ionentransportstrecke des zweiten Ionenstrahlsystems vorgehalten ist.

Unter der Vorgabe eine Ionenstrahlanlage a priori auch für einen späteren Betrieb mit schwereren Ionen zu rüsten, könnte an einen Ersatz der Komponenten des ersten Ionenstrahlsystems durch die wesentlich größeren Komponenten des zweiten Ionenstrahlsystems gedacht werden. Dadurch würde aber mit Nachteil das anfängliche Investment aufgrund der entsprechend überdimensioniert vorzuhaltenden Räume belastet, und der Ersatz der Komponenten würde darüber hinaus mit Nachteil zu langen Stillstandszeiten der Ionenstrahlanlage führen. Eine von Anfang an "überdimensioniert" Ausbildung der Komponenten des ersten Ionenstrahlsystems für einen späteren Betreib auch mit schwereren Ionen würde zwar vorgenannte lange Stillstandszeiten verhindern, würde aber mit Nachteil das anfängliche Investment aufgrund der teuren "überdimensionierten" Komponenten neben den großen Räumen zusätzlich belasten. Dahingegen werden vorgenannte Nachteile bei den Lösungen gemäß den Ansprüchen 1 und 2 vermieden, so dass das Investment für das erste Ionenstrahlsystem und dessen Gebäude weder durch übergroße Räume noch "überdimensionierte" Komponenten des Ionenstrahlsystems belastet wird.

Auch bei der Ionenstrahlanlage gemäß Anspruch 6, bei der wenigstens einer der ersten Bestrahlungsplätze des ersten Ionenstrahlsystems auch am zweiten Ionenstrahlsystem betreibbar ist, wie auch bei der Ionenstrahlanlage nach Anspruch 8, bei der die beiden Ionentransportstrecken des ersten und zweiten Ionenstrahlsystems getrennt durch eine Wand parallel geführt sind, sind vorgenannte Vorteile erzielbar, wobei durch die Parallelführung weiterhin ein kompakter Gesamtaufbau der Ionenstrahlanlage erreicht wird.

Weitere Vorteile, Merkmale und Einzelheiten der Erfindung ergeben sich aus den nachfolgend beschriebenen Ausführungsbeispielen der Erfindung anhand der Figuren. Dabei zeigen:
Figur 1 ein erstes Ionenstrahlsystem in einer perspektivischen Ansicht,
Figur 2 eine Ionenstrahlanlage in einer ersten Ausbaustufe mit dem Ionenstrahlsystem der Figur 1 in grundrissartiger Darstellung,
Figur 3 einen Querschnitt der Ionenstrahlanlage der Figur 2 in einer ersten Ausführungsform,
Figur 4 einen Querschnitt der Ionenstrahlanlage der Figur 2 in einer zweiten Ausführungsform,
Figur 5 die um ein zweites Ionenstrahlsystem in einer ersten Ausführungsform erweiterte Ionenstrahlanlage der Figur 2 in grundrissartiger Darstellung,
Figur 6 die um ein zweites Ionenstrahlsystem in einer zweiten Ausführungsform erweiterte Ionenstrahlanlage der Figur 2 in grundrissartiger Darstellung und
Figur 7 eine weitere Ionenstrahlanlage mit einem ersten und einem zweiten Ionenstrahlsystem in grundrissartiger Darstellung.

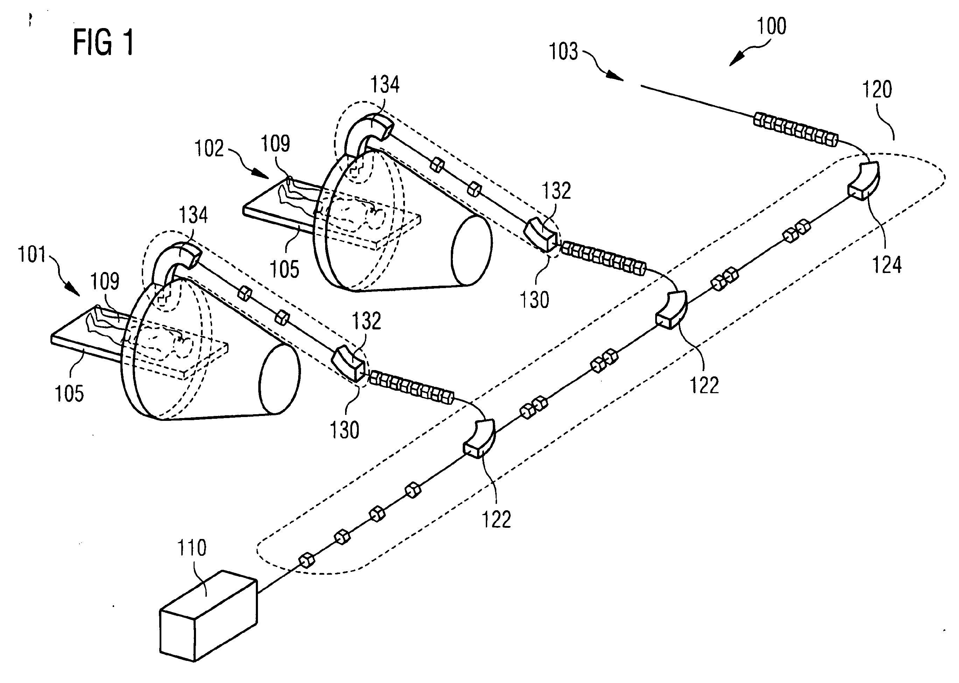

Die Figur 1 zeigt als einen Bestandteil einer als Ausführungsbeispiel der Erfindung dienenden Ionenstrahlanlage ein erstes Ionenstrahlsystem 100 zur Bestrahlung von Patienten 109 mit Ionen einer ersten Ionenart, insbesondere mit Protonen, die auch als Wasserstoffionen bezeichnet werden. Dabei umfasst das erste Ionenstrahlsystem 100 einen Ionenstrahlerzeuger 110, umfassend eine Ionenquelle und einen Ionenbeschleuniger, der mit einer Ionentransportstrecke 120 verbunden ist. Über die Ionentransportstrecke 120 werden die hochenergetischen Ionen zu den Patienten 109 an einem auswählbaren der Bestrahlungsplätze 101, 102 und 103 geführt. Dazu beinhaltet die Ionentransportstrecke 120 zwei Ionenstrahlweichen 122 und einen Ionenstrahlumlenker 124. Jede der Ionenstrahlweichen 122 ist dabei durch zwei Schaltzustände gekennzeichnet. In einem ersten Schaltzustand wird der vom Ionenstrahlerzeuger 110 herkommende Ionenstrahl in Richtung eines der Bestrahlungsplätze 101 oder 102 gelenkt und in einem zweiten Schaltzustand vermag der Ionenstrahl die jeweilige Ionenstrahlweiche 122 geradlinig zu durchdringen.

Der Bestrahlungsplatz 103 ist derart ausgebildet, dass der Ionenstrahl fest vorgegebenen in einer horizontalen Richtung austritt. Die beiden anderen Bestrahlungsplätze 101 und 102 umfassen jeweils eine, um eine horizontale Achse drehbare Ionenstrahlführvorrichtung 130, die den Ionenstrahl in Richtung der Achse aufnimmt, ihn weg von der Achse transportiert und ihn rechtwinkelig zur Achse, die Achse schneidend, ausrichtet. Dabei umfasst die Ionenstrahlführvorrichtung 130 unter anderem einen 45°-Ionenstrahlumlenker 132 und einen 135°-Ionenstrahlumlenker 134. Der Schnittpunkt des Ionenstrahls mit der Achse stellt dabei das Isozentrum eines Zielgebiets in einem der Patienten 109 dar, der in einer vorgebbaren Richtung auf einer Patientenlagerungsvorrichtung 105 gelagert ist. Dabei vermag vorgenannte drehbare Ionenstrahlführvorrichtung 130 während der Bestrahlung eines der Patienten 109 durch ein Verschwenken der Strahlenführvorrichtung 130 den Ionenstrahl von unterschiedlichen Winkeln her durch das Isozentrum zu lenken.

Bei dem Ionenstrahlsystem 100 sind dabei konventionelle Bauelemente entsprechend wohlbekannter Transport-, Beschleunigungs- und Fokussierungstechniken für Ionen genutzt, kombiniert, aufeinander abgestimmt und derart eingestellt, dass die gewünschten Beschleunigungs- und Injektionsparameter erzielt werden.

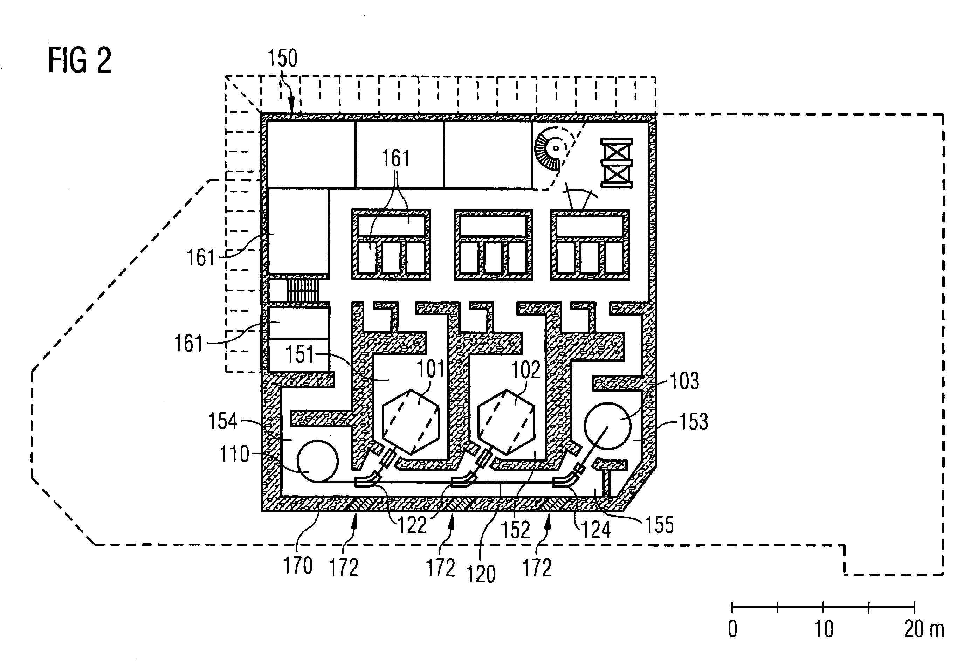

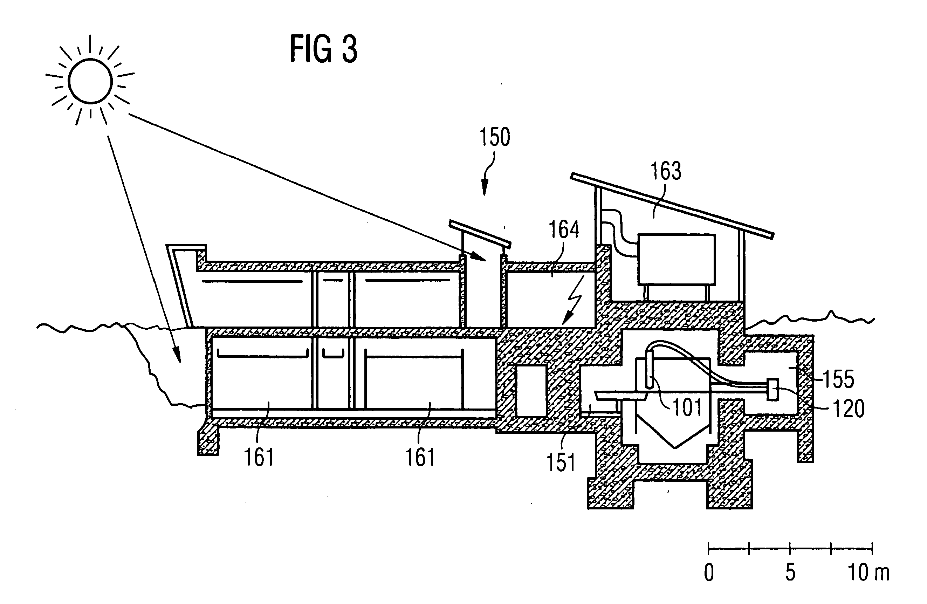

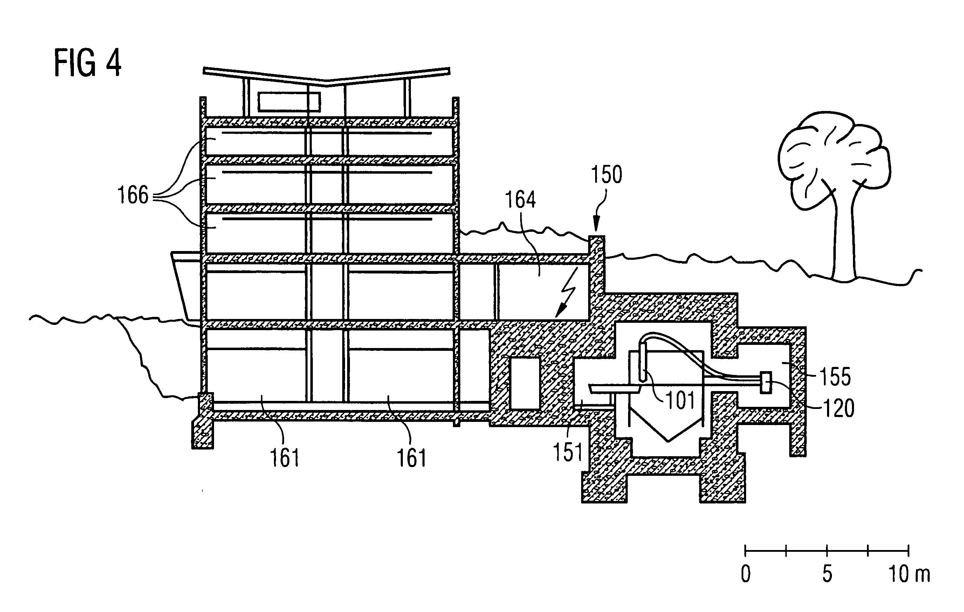

Die Figur 2 zeigt als ein Ausführungsbeispiel der Erfindung die Ionenstrahlanlage in einer ersten Ausbaustufe. Dabei ist das Ionenstrahlsystem 100 der Figur 1 in einem Gebäude 150 angeordnet, dessen Grundriss in der Figur 2 skizziert ist. Die Ionenstrahlstrecke 120 ist dabei entlang einer Strahlung dämmenden Wand 170 des Gebäudes 150 geführt, die in der ersten Ausbaustufe gleichzeitig eine Außenwand des Gebäudes 150 darstellt. Die Bestrahlungsplätze 101, 102 und 103, der Ionenstrahlerzeuger 110 und die Ionentransportstrecke 120 sind dabei in voneinander gegen Strahlung gedämmten Räumen 151 bis 155 des Gebäudes 150 unterbebracht. Dabei ist die Strahlendämmung durch Betonwände mit wenigen Metern Dicke sichergestellt. Die Strahlendämmung ist notwendig, da bei der Interaktion des Ionenstrahles mit Materie, beispielsweise mit der Luft, mit Gewebe, mit Geräten zur Messung von Strahlparametern im Ionenstrahlerzeuger 110 und/oder der Ionenstrahltransportstrecke 120 usw., ionisierende Sekundärstrahlung entsteht, die überwiegend schnelle Neutronen beinhaltet, deren Energie ausreicht, menschliche Zellen zu schädigen. Zum Schutz von Personal und Öffentlichkeit vor dieser Strahlung sind die Räume 151 bis 155 durch Mauern aus Beton bzw. wie in den Figuren 3 und 4 dargestellt durch den angrenzenden Erdboden gedämmt. Die Strahlendämmung muss unter anderem in Abhängigkeit von der Ionenart in ihrer Dicke dergestalt bemessen sein, dass das Strahlungsniveau jenseits der Dämmung auf ein akzeptables, vom Gesetzgeber vorgeschriebenes Niveau abgesenkt ist. Weitere Räume 161 des Gebäudes 150 stehen zur Betriebsüberwachung der Anlage, zur Behandlung und Betreuung von Patienten 109 sowie für damit zusammenhängende Bürotätigkeiten zur Verfügung.

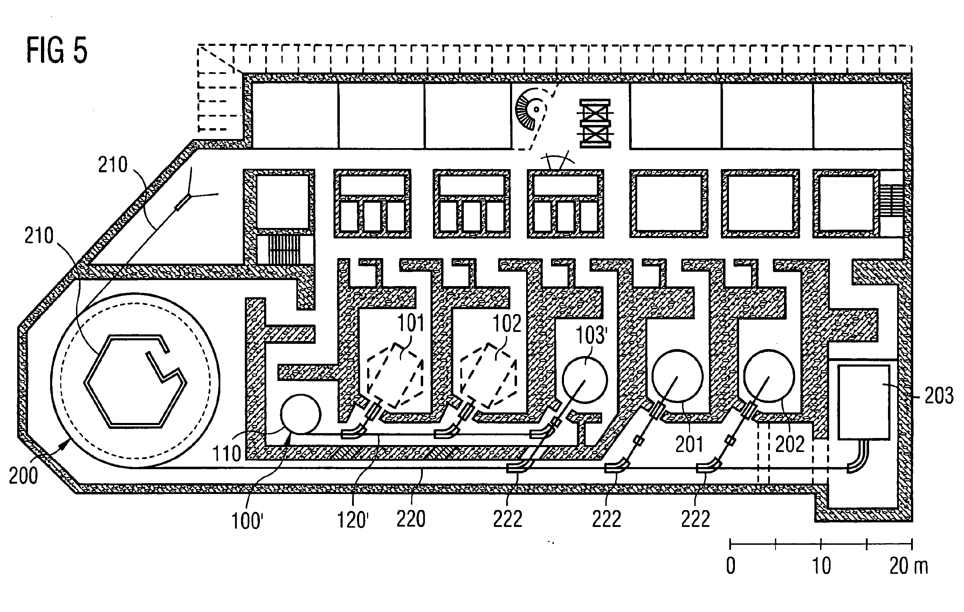

Die Ionenstrahlanlage ist in der ersten Ausbausstufe bereits derart konzipiert und hergerichtet, dass sie ohne eine Betriebsunterbrechung des Ionenstrahlsystems 100 um ein zweites Ionenstrahlsystem 200 für eine zweite Ionenart, die durch schwerere Ionen als die erste Ionenart, beispielsweise Kohlenstoffionen, gekennzeichnet ist, erweiterbar ist. Für diese Erweiterung ist dabei der mit gestrichelter Linie gezeichnete Grundriss für einen gebäudetechnischen Ausbau vorgehalten. Ferner ist die Wand 170 bereits derart für Öffnungen 172 hergerichtet, so dass im Zuge der Erweiterung wenigstens einzelne der Bestrahlungsplätze 101, 102 und 103 mit geringem Zeitaufwand und damit ohne größere Betriebsunterbrechungen für einen Betrieb ausschließlich mit dem zweiten Ionenstrahlsystem 200 oder für einen Mischbetrieb im Rahmen beider Ionenstrahlsystem 100 und 200 umrüstbar sind. Für die Öffnungen 172 kann dabei die Wand 170 beispielsweise mit entsprechenden Ausmauerungen versehen sein. Bis zu einem Erweitern der Ionenstrahlanlage sind die Öffnungen 172 mit entsprechenden Verschlüssen dicht verschlossen, die hinsichtlich der Strahlendämmung mit entsprechenden Eigenschaften wie die Wand 170 ausgebildet sind. Weiteres zu vorgenannter Erweiterung ist nachfolgend bei der Figur 5 beschrieben.

Die Figur 3 zeigt einen Querschnitt durch die in der Figur 2 als Grundriss dargestellte Ionenstrahlanlage, wobei der Querschnitt in etwa durch die Räume 155 und 151 geführt ist. Dabei ist das Gebäude 150 derart gestaltet, dass wenigstens die strahlungsgedämmten Räume 151 bis 155 aus Gründen eines möglichst guten und einfachen zusätzlichen Strahlenschutzes mit ihrer Deckenoberkante in etwa entsprechend der Erdbodenoberfläche übereinstimmen, so dass der Erdboden zusätzlich als Strahlenschutz genutzt wird. In einem weiteren Geschoss über den Räumen 151 bis 161 sind beispielsweise ein Raum 163 für elektrische Betriebseinrichtungen sowie ein Raum 164 für Klimatisierungseinrichtungen, insbesondere für die Räume 151 bis 155, angeordnet.

Die Figur 4 zeigt einen weiteren Querschnitt durch die Ionenstrahlanlage der Figur 2. Gegenüber der Figur 3 sind zusätzliche Geschossaufbauten, beispielsweise für weitere krankenhaustechnische Funktionen, mit den Räumen 166 vorgesehen, und kein spezieller Raum 164 für Klimatisierungseinrichtungen vorgesehen.

Die Figur 5 zeigt schließlich ausgehend von der Figur 2 die Ionenstrahlanlage nach Erweiterung um das zweite Ionenstrahlsystem 200. Dabei umfasst das zweite Ionenstrahlsystem 200 ähnlich wie das erste Ionenstrahlsystem 100, einen Ionenstrahlerzeuger 210, eine Ionentransportstrecke 220 mit entsprechenden Ionenstrahlweichen 222, und drei Bestrahlungsplätze 201, 202 und 203. Dabei weisen die Bestrahlungsplätze 201 und 202 einen fest vorgegebenen horizontalen Ionenstrahlaustritt auf, wohingegen der Bestrahlungsplatz 203 entsprechend dem im Artikel von S. Reimoser et al. "Engineering design and study of a beam position accuracy in the "Riesenrad" ion gantry", Nuclear Instruments and Methods in Physics Research A 456 (2001), Seiten 390 bis 410, beschriebenen Bestrahlungsplatz ausgebildet ist. Insbesondere der Ionenstrahlerzeuger 210 und auch eine drehbare Ionenstrahlführvorrichtung für den Bestrahlungsplatz 203 sind dabei aufgrund der Betriebsmöglichkeit mit den schwereren Ionen wesentlich voluminöser als die vergleichbaren Komponenten für die ersten leichteren Ionen, so dass insbesondere die Räume für den Ionenstrahlerzeuger 210 und den Bestrahlungsplatz 203 deutlich mehr Platz beanspruchen als beim ersten Ionenstrahlsystem 100.

Die Ionentransportstrecke 220 ist entlang der Wand 170 geführt. Dies hat insbesondere den Vorteil, dass die Bestrahlungsplätze 103, 102 und/oder 101 des ersten Ionenstrahlsystems 100 für den Mischbetrieb mit beiden Ionenstrahlsystemen 100 und 200 oder einen ausschließlichen Betrieb mit dem zweiten Ionenstrahlsystem 200 einfach umrüstbar sind, wobei für eine einfache Umrüstung und kurze Nutzungsausfälle der Bestrahlungsplätze 103, 102 und/oder 101 entweder nur der Bestrahlungsplatz 103 oder zunächst der Bestrahlungsplatz 103 und nachfolgend der Bestrahlungsplatz 102 oder beginnend mit dem Bestrahlungsplatz 103, dann der Bestrahlungsplatz 102 und schließlich der Bestrahlungsplatz 101 umgerüstet werden. Für die Erweiterung ist die Ionentransportstrecke 220 unter anderem durch den Einsatz von Ionenstrahlweichen 222 entsprechend hergerichtet. Für die Umrüstung sind dann die in der Wand 170 vorgesehenen Öffnungen 172 entsprechend freizumachen und die Bestrahlungsplätze 103, 102 und/oder 101 sowie die Ionentransportstrecke 120 entsprechend umzurüsten bzw. anzupassen, wobei insbesondere die Strahlendämmung der Räume 153, 152 und/oder 151 für den Betrieb mit den schwereren Ionen zu ertüchtigen ist, indem beispielsweise die Wände durch Fertigteile in Form von Betonblöcken entsprechend verstärkt werden. Da das zweite Ionenstrahlsystem 200 vom ersten Ionenstrahlsystem 100 strahlungstechnisch entkoppelt ist, kann die Erweiterung und Umrüstung der Ionenstrahlanlage weitgehend bei weitergeführtem Betrieb des ersten Ionenstrahlsystems 100 durchgeführt werden. Des Weiteren ist vorteilhaft, dass bei dem Investment für das erste Ionenstrahlsystem 100 und dessen Gebäude 150 zunächst keine zusätzlichen Kosten für vergrößerte Räume entstehen, wie dies der Fall wäre, wenn man an Stelle der beschriebenen Erweiterung an einen Ersatz der Komponenten des ersten Ionenstrahlsystems 100 durch Komponenten des zweiten Ionenstrahlsystems 200 denken würde, was darüber hinaus mit Nachteil mit sehr langen Stillstandszeiten der Ionenstrahlanlage verbunden wäre.

In der Figur 5 ist dabei exemplarisch der Bestrahlungsplatz 103' ausgehend von dem Bestrahlungsplatz 103 für den Mischbetrieb umgerüstet. Dazu umfasst die Ionentransportstrecke 220 an entsprechender Stelle eine Ionenstrahlweiche 222. Ferner ist ausgehend von der Ionentransportstrecke 120 die Ionentransportstrecke 120' als Bestandteil eines angepassten ersten Ionenstrahlsystems 100' im rechten Endbereich entsprechend angepasst. Schließlich ist gegenüber dem Raum 153 für den Bestrahlungsplatz 103 der Raum für den Bestrahlungsplatz 103' umgestaltet, indem beispielsweise für den Strahlenschutz Wände verdickt sind. Der Bestrahlungsplatz 103' weist dabei den besonderen Vorteil auf, dass im Falle einer Wartung des angepassten ersten Ionenstrahlsystems 100' oder des zweiten Ionenstrahlsystems 200 mit dem zweiten Ionenstrahlsystem 200 oder dem angepassten ersten Ionenstrahlsystem 100' weiter Betrieb gemacht werden kann, wobei insbesondere bei Wartung des angepassten ersten Ionenstrahlsystems 100' durch Umjustierungen des zweiten Ionenstrahlsystems 200 dieses durchaus auch mit Ionen der ersten Ionenart betreibbar ist.

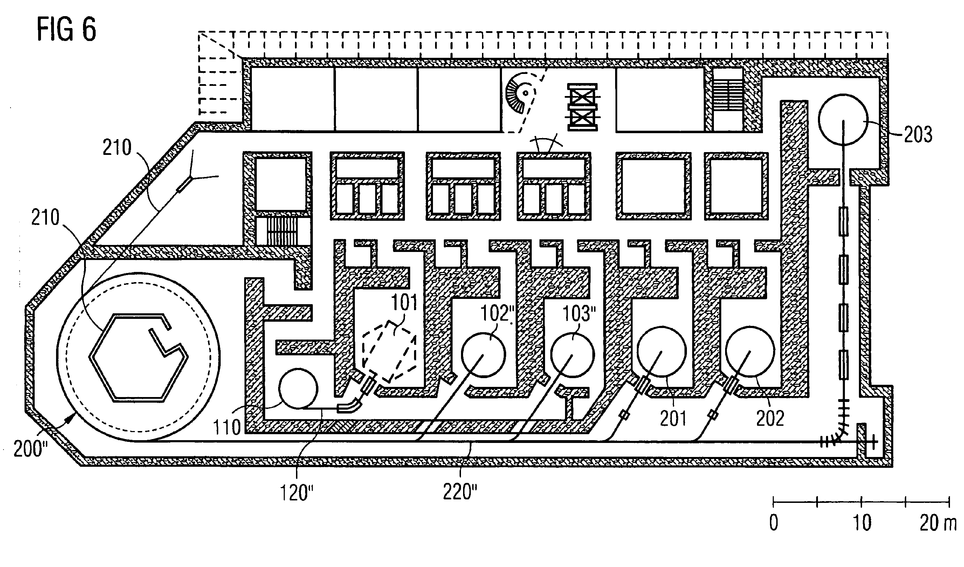

Die Figur 6 zeigt als ein weiteres Ausführungsbeispiel der Erfindung eine andere Ausführungsform der Erweiterung. Gegenüber der Figur 5 wird bei der Figur 6 kein Mischbetrieb ermöglicht. Ausgehend von der Figur 2 sind die Bestrahlungsplätze 102 und 103 zu ausschließlich mit einem zweiten Ionenstrahlsystem 200'' betreibbaren Bestrahlungsplätzen 102'' und 103'' mit fest vorgegebenem Strahlaustrittswinkel umgerüstet. Die Ionentransportstrecke 120 ist zur ausschließlichen Versorgung des Bestrahlungsplatzes 101 zur verkürzten Ionentransportstrecke 120'' umgerüstet. Die Bestrahlungsplätze 102'', 103'', 201, 202 und 203'' sind dabei an einer Ionentransportstrecke 220'' des Ionenstrahlsystems 200'' angeschlossen. Der dem Bestrahlungsplatz 203'' unmittelbar vorgeschaltete, in der Figur 6 vertikal verlaufende Teil der Ionentransportstrecke 220'' ist dabei derart ausgestaltet, dass am Bestrahlungsplatz 203'' bezüglich einer zum Raumboden parallelen Patientenlagerfläche der Ionenstrahl in einem 60°-Winkel von oben her austritt.

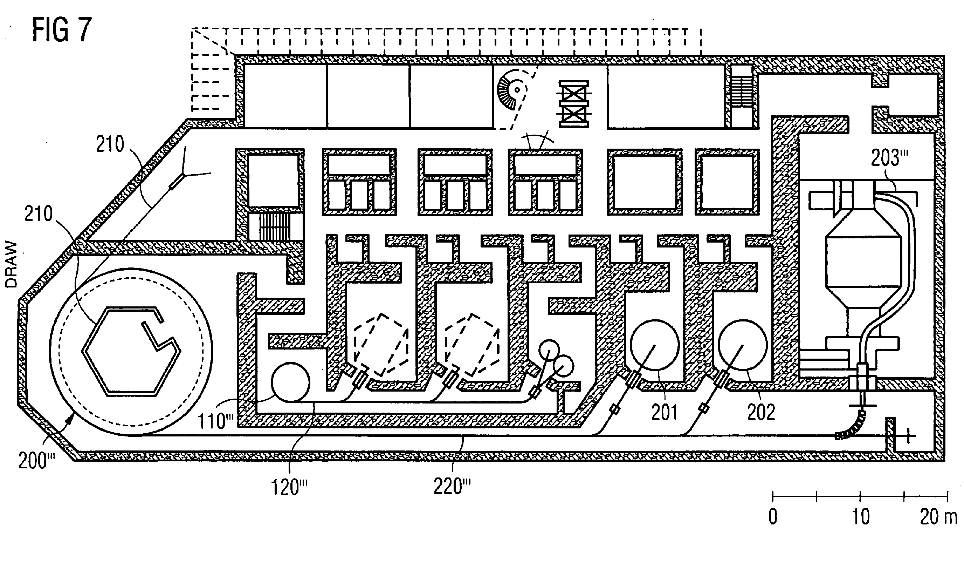

Die Figur 7 zeigt als ein weiteres Ausführungsbeispiel der Erfindung eine Ionenstrahlanlage, bei der ein erstes Ionenstrahlsystem 100''' für leichte Ionen, das im Wesentlichen dem ersten Ionenstrahlsystem 100 entspricht, mit einem zweiten Ionenstrahlsystem 200''' für schwere Ionen parallel betrieben wird. Dabei umfasst das Ionenstrahlsystem 200''' den Ionenstrahlerzeuger 210, eine Ionentransportstrecke 220''' sowie Bestrahlungsplätze 201, 202 und 203''', wobei der Bestrahlungsplatz 203''' entsprechend den Bestrahlungsplätzen 101 und 102 mit einer drehbaren Ionenstrahlführvorrichtung und Dimensionierung für die schweren Ionen ausgebildet ist. Durch die Parallelführung der beiden Ionentransportstrecken 220''' und 120''' beiderseits einer Strahlung dämmenden Wand 170''' entsteht ein kompakter Gesamtaufbau der Ionenstrahlanlage.

In einer anderen Ausführungsform kann natürlich auch die Ionenstrahlanlage der Figur 7 aus einer Erweiterung der in der Figur 2 beschriebenen Ionenstrahlanlage mit den vorausgehend beschriebenen Vorteilen hervorgehen. Ebenso müssen natürlich auch die bei den Figuren 5 und 6 beschriebenen Ionenstrahlanlagen nicht zwangläufig aus der beschriebenen Erweiterung resultieren, sondern können von Anfang an so errichtet werden, wobei aufgrund der Parallelführung der Ionentransportstrecken der beiden Ionenstrahlsysteme dann nach wie vor mit Vorteil vorausgehend beschriebene einfache Umrüstmöglichkeiten von Bestrahlungsplätzen für das eine oder andere Ionenstrahlsystem oder den Mischbetrieb gegeben sind.

## Patentansprüche

1. Ionenstrahlanlage mit einem Gebäude mit mindestens einer Wand, beinhaltend folgende Merkmale:
- Ein erstes Ionenstrahlsystem für eine erste Ionenart, das einen ersten Ionenstrahlerzeuger und eine erste Ionentransportstrecke mit wenigstens einer ersten Ionenstrahlweiche für einen von wenigstens zwei ersten Bestrahlungsplätzen der Ionenstrahlanlage beinhaltet,
- die erste Ionentransportstrecke ist entlang der Wand des Gebäudes der Ionenstrahlanlage geführt,
**dadurch gekennzeichnet, dass**
- die Wand für mindestens eine Öffnung mittels einer mit einem Verschluss dicht verschlossenen Ausmauerung oder mit einer Sollbruchstelle derart vorbereitet ist, dass über eine auf der anderen Seite der Wand nachinstallierbare zweite Ionentransportstrecke eines zweiten Ionenstrahlsystems für eine zweite Ionenart wenigstens einer der ersten Bestrahlungsplätze auch am zweiten Ionenstrahlsystem betreibbar ist, indem der Verschluss entfernbar ist.

2. Ionenstrahlanlage nach Anspruch 1,
**dadurch gekennzeichnet, dass** die Wand eine Außenwand des Gebäudes ist.

3. Ionenstrahlanlage nach einem der Ansprüche 1 oder 2,
**dadurch gekennzeichnet, dass** die beiden Ionentransportstrecken zueinander im Wesentlichen parallel verlaufend anordenbar sind.

4. Ionenstrahlanlage nach einem der Ansprüche 1 bis 3,
**dadurch gekennzeichnet, dass** das Gebäude für eine Erweiterung um das zweite Ionenstrahlsystem beiderseits einer durch die erste Ionentransportstrecke definierten Richtung erweiterbar angelegt ist.

5. Ionenstrahlanlage nach einem der Ansprüche 1 bis 4,
**dadurch gekennzeichnet, dass** die Wand die Strahlung dämmend ausgebildet ist.

6. Ionenstrahlanlage nach einem der Ansprüche 1 bis 5,
**dadurch gekennzeichnet, dass** die Wand Beton beinhaltet und eine Dicke von wenigen Metern aufweist.

7. Ionenstrahlanlage nach einem der Ansprüche 1 bis 6,
**dadurch gekennzeichnet, dass** die erste Ionenstrahlstrecke im Wesentlichen geradlinig ausgebildet ist.

8. Ionenstrahlanlage nach einem der Ansprüche 1 bis 7,
**dadurch gekennzeichnet, dass** eine Decke der Räume der Bestrahlungsplätze und/oder der Ionenstrahlerzeuger in etwa mit der Erdbodenoberfläche niveaugleich ist.

9. Ionenstrahlanlage nach einem der Ansprüche 1 bis 8,
**dadurch gekennzeichnet, dass** wenigstens einer der Bestrahlungsplätze für einen Austritt des Ionenstrahls in einer fest vorgebbaren Richtung ausgebildet ist.

10. Ionenstrahlanlage nach einem der Ansprüche 1 bis 9,
**dadurch gekennzeichnet, dass** wenigstens einer der Bestrahlungsplätze mit einer drehbaren Ionenstrahlführvorrichtung ausgebildet ist, so dass eine Austrittsrichtung des Ionenstrahls um wenigstens eine Achse drehbar ist.

## Claims

1. Ion beam facility with a building having at least one wall, comprising the following features:
- a first ion beam system for a first ion type having a first ion beam generator and a first ion transport line comprising at least one first ion beam switch for one of at least two first irradiation stations of the ion beam facility,
- the first ion transport line is conducted along the wall of the building of the ion beam facility,
**characterised in that**
- the wall is prepared for at least one opening by means of a cladding which is tightly sealed with a seal or with a rated breaking point such that at least one of the first irradiation stations can also be operated on the second ion beam system by way of a second ion transport line of a second ion beam system for a second ion type, said second ion transport line being subsequently installed on the other side of the wall, by the seal being removable.

2. Ion beam facility according to claim 1,
**characterised in that**
the wall is an outside wall of the building.

3. Ion beam facility according to one of claims 1 or 2,
**characterized in that**
the two ion transport lines can be arranged essentially parallel to one another.

4. Ion beam facility according to one of claims 1 to 3,
**characterized in that**
the building is designed to be expandable for an expansion by the second ion beam system on both sides of a direction defined by the first ion transport line.

5. Ion beam facility according to one of claims 1 to 4,
**characterized in that**
the wall is designed to be a radiation-blocking wall.

6. Ion beam facility according to one of claims 1 to 5,
**characterized in that**
said wall contains concrete and has a thickness of several meters.

7. Ion beam facility according to one of claims 1 to 6 **characterized in that** the first ion transport line proceeds substantially along a straight line.

8. Ion beam facility according to one of claims 1 to 7,
**characterized in that**
a ceiling of the rooms of the irradiation stations and/or the ion beam generator is approximately at ground level.

9. Ion beam facility according to one of claims 1 to 8,
**characterized in that**
at least one of the irradiation stations is designed to allow an output of ions in a rigidly prescribed direction.

10. Ion beam facility according to one of claims 1 to 9,
**characterised in that**
at least one of the irradiation stations is designed with a rotatable ion beam guidance mechanism, allowing a rotation of an output of ions around at least one axis.

## Revendications

1. Installation à faisceau ionique comportant un bâtiment ayant au moins un mur et comportant les caractéristiques suivantes :
- un premier système à faisceau ionique pour un premier type d'ions, qui comporte un premier générateur de faisceau ionique et une première trajectoire de transport des ions ayant au moins une première bifurcation du faisceau ionique pour un poste d'irradiation parmi au moins deux premiers postes d'irradiation de l'installation à faisceau ionique,
- la première trajectoire de transport des ions passe le long du mur du bâtiment de l'installation à faisceau ionique,
**caractérisée en ce que**
- le mur, pour au moins une ouverture, est préparé au moyen d'une fausse paroi fermée de manière étanche par un système d'obturation ou au moyen d'une zone destinée à se rompre, de telle sorte que, par l'intermédiaire d'une deuxième trajectoire de transport des ions d'un deuxième système à faisceau ionique, pouvant être mis en place ultérieurement de l'autre côté du mur, pour un deuxième type d'ions, au moins l'un des premiers postes d'irradiation peut fonctionner également sur le deuxième système à faisceau ionique, du fait que le système d'obturation peut être retiré.

2. Installation à faisceau ionique selon la revendication 1, **caractérisée en ce que** le mur est un mur extérieur du bâtiment.

3. Installation à faisceau ionique selon la revendication 1 ou 2, **caractérisée en ce que** les deux trajectoires de transport des ions peuvent être disposées en s'étendant sensiblement parallèlement l'une à l'autre.

4. Installation à faisceau ionique selon l'une quelconque des revendications 1 à 3, **caractérisée en ce que** le bâtiment est réalisé de manière extensible pour un agrandissement avec le deuxième système à faisceau ionique de part et d'autre d'une direction définie par la première trajectoire de transport des ions.

5. Installation à faisceau ionique selon l'une quelconque des revendications 1 à 4, **caractérisée en ce que** le mur est réalisé de manière à atténuer le rayonnement.

6. Installation à faisceau ionique selon l'une quelconque des revendications 1 à 5, **caractérisée en ce que** le mur contient du béton et a une épaisseur de quelques mètres.

7. Installation à faisceau ionique selon l'une quelconque des revendications 1 à 6, **caractérisée en ce que** la première trajectoire de transport des ions est sensiblement rectiligne.

8. Installation à faisceau ionique selon l'une quelconque des revendications 1 à 7, **caractérisée en ce qu'**un plafond des postes d'irradiation et/ou des générateurs de faisceau ionique est situé à peu près au même niveau que la surface du sol.

9. Installation à faisceau ionique selon l'une quelconque des revendications 1 à 8, **caractérisée en ce qu'**au moins l'un des postes d'irradiation est réalisé pour une sortie du faisceau ionique dans une direction prédéfinissable de manière fixe.

10. Installation à faisceau ionique selon l'une quelconque des revendications 1 à 9, **caractérisée en ce qu'**au moins un des postes d'irradiation est réalisé en ayant un dispositif rotatif de guidage du faisceau ionique, de telle sorte qu'une direction de sortie du faisceau ionique puisse tourner autour d'au moins un axe.
